# FASCICULE DE BREVET EUROPEEN

(11) **EP 0 974 645 B1**
(45) Date de publication et mention de la délivrance du brevet: **02.10.2002**
(21) Numéro de dépôt: 98401784.8
(22) Date de dépôt: 15.07.1998
(51) Int. Cl.: C12N 1/20, C12N 5/00

(54) **Procédé de préparation par les microondes de milieux de cultures stériles, liquides et solides, pour la sélection et l'identification de microorganismes recombinants**
Verfahren zur Herstellung von sterilen flüssigen oder festen Kulturmedien mittels Mikrowellen, zur Erkennung und Identifizierung von rekombinanten Mikroorganismen
Procedure to prepare, by the use of microwaves, sterile liquid or solid culture media, for the recognition and identification of recombinant microorganisms

(43) Date de publication de la demande: 26.01.2000
(73) Titulaire: CAYLA, 31400 Toulouse (FR)
(72) Inventeur: Armau, Elise, 31400 Toulouse (FR); Tiraby, Gérard, 31400 Toulouse (FR)
(74) Mandataire: Boulinguiez, Didier

(56) Documents cités:
- FR-A- 2 747 394
- US-A- 5 500 367
- US-A- 5 674 730
- BAQAI ET AL.: "Microwave oven in microbiology laboratory" THE JOURNAL OF THE PAKISTAN MEDICAL ASSOCIATION, vol. 42, no. 1, janvier 1992, pages 2-3, XP002088158
- KELLER ET AL.: "Microwave treatment for sterilization of phytoplankton culture media" JOURNAL OF EXPERIMENTAL MARINE BIOLOGY AND ECOLOGY, vol. 117, no. 3, 1988, pages 279-283, XP002088159
- TISSERAT ET AL.: "Microwave sterilization of plant tissue culture media" HORTSCIENCE, vol. 27, no. 4, 1992, pages 358-361, XP002088160

## Description

Les continuelles avancées de la biologie moléculaire des acides nucléiques (ADN et ARN) ont grandement contribué à l'évolution des connaissances dans tous les domaines de la biologie. Toutes les disciplines biologiques traditionnelles confondues ont bénéficié de cet apport moléculaire. On peut citer la microbiologie et ses sous-branches que sont la bactériologie, la mycologie, la parasitologie et la virologie, mais aussi la biologie cellulaire, la physiologie cellulaire, la génétique moléculaire, la biochimie, l'enzymologie, l'immunologie et la physiologie animale. L'exploitation des potentialités des acides nucléiques a abouti à l'apparition de nouveaux domaines d'investigation hier encore inconnus désignés par des termes nouveaux tels que la génomique, la médecine moléculaire ou la thérapie génique.

Les technologies de l'ADN recombinant ont pu évoluer aussi rapidement grâce à l'utilisation de la bactérie *Escherichia coli*. La souche K12 d'*Escherichia coli* est devenue avec ses nombreux mutants, au fil des années, un intermédiaire incontournable pour toute opération en rapport avec les acides nucléiques. Tout expérimentateur manipulant de l'ADN recombinant dans pratiquement tous les domaines de la biologie, est amené à travailler avec cette bactérie notamment sous la forme dite transformée du fait de l'introduction dans la cellule d'ADN plasmidique ou viral. C'est ainsi que par exemple les préparations d'ADN recombinés, pouvant renfermer des fragments d'ADN naturels ou modifiés de toutes origines pris parmi le monde des archéons, procaryotes et eucaryotes et même synthétiques, sont faites dans leur très grande majorité à partir de vecteurs spécifiques à *Escherichia coli*. Les souches de laboratoire de la levure *Saccharomyces cerevisiae* doivent aussi être mentionnées comme faisant partie des microrganismes les plus employés pour la production d'ADN recombinant.

La transformation d'une bactérie ou d'un microorganisme eucaryote par un vecteur est une opération couramment pratiquée par les expérimentateurs qui se déroule en deux étapes. La première consiste à introduire l'ADN du vecteur dans les cellules au moyen de diverses techniques et la seconde à sélectionner les cellules de la population qui ont recu et expriment les gènes du vecteur. En effet, la fraction des cellules qui expriment les gènes du vecteur par rapport au nombre total des cellules varie fortement en fonction de la qualité et du type de l'ADN transformant et du protocole de transfert mis en oeuvre. Même dans le meilleur des cas, la population transformée des cellules est toujours faible. Aussi plusieurs moyens ont été imaginés pour ne sélectionner que les cellules exprimant le ou les gènes nouveaux apportés par l'ADN transformant. Tous reposent sur l'expression d'un gène dont le produit apportera un caractère dominant à la cellule par rapport aux cellules non transformées. C'est par exemple l'utilisation d'un gène de résistance à un antibiotique normalement toxique pour le microorganisme récepteur. L'addition d'un antibiotique donné à une culture microbienne ayant été mise en contact avec l'ADN porteur du gène de résistance à cet antibiotique bloquera le développement ou tuera suivant la nature de l'antibiotique les cellules n'exprimant pas le transgène alors qu'au contraire les cellules transformées devenues résistantes se multiplieront pour finalement aboutir à une culture pure constituée de cellules résistantes. Pour des raisons pratiques la sélection se fait le plus souvent avec des milieux solides en boite de Petri. Dans ce cas chaque cellule transformée donnera une culture pure indépendante qui se traduira par la formation de colonies à la surface du milieu contenu dans la boite de Petri. Les antibiotiques les plus employés pour la sélection des clones transformés chez *E.coli* sont l'ampicilline et la carbenicilline parmi les pénicillines, la kanamycine et la néomycine parmi les aminosides, la tetracycline, le chloramphénicol et la zéocine.

L'utilisation de marqueurs de sélection est souvent associée avec l'emploi de marqueurs d'identification c'est à dire des gènes qui permettent de distinguer les colonies transformées des colonies n'exprimant pas le transgène en question par une coloration différente. Le système le plus populaire parmi les expérimentateurs est celui basé sur l'expression du gène lacZ de la β-galactosidase d'*Escherichia coli*. L'action de cette enzyme dans son intégralité ou reconstituée comme dans le système de l'alpha peptide exploité dans beaucoup de vecteurs de clonage chez *E.coli* conduit à l'hydrolyse d'un composé incolore le 5-bromo-4-chloro-3-indoxyl-β-D-galactopyranoside (ou X-gal ) pour donner un produit insoluble bleu. La présence de ce colorant dans les milieux solides pour *E.coli* par exemple conduira à l'apparition de colonies bleues formées par des cellules produisant une β-galactosidase active alors que les cellules ne possédant pas de β-galactosidase donneront des colonies blanches. De nombreux autres substrats chromogéniques ou fluorescents de la β-galactosidase sont utilisés pour une même raison de distinction visuelle des colonies positives parmi une population de colonies blanches ou non fluorescentes. Des substrats similaires chromogéniques ou fluorescents sont aussi couramment utilisés pour la détection de colonies positives pour d'autres activités enzymatiques telles que la β-glucuronidase et la phosphatase alcaline par exemple.

Le clonage de gènes dans des vecteurs d'*E. coli* ne demande plus un niveau de qualification nécessitant plusieurs années d'expérience comme c'était encore le cas dans les années 80. L'acquisition de ces technologies les plus courantes commence déja bien souvent au niveau de la formation scolaire et se poursuit pendant les cours de biologie moléculaire à l'université dans tout pays à forte culture scientifique. Les méthodologies en relation avec la manipulation de l'ADN ou l'ARN se sont beaucoup simplifiées par l'introduction successive d'améliorations conçues par la communauté scientifique entière, divulguées par l'intermédiaire des publications. Un autre élement décisif qui a conduit à la simplification mais aussi au raccourcissement du temps nécessaire pour réaliser une expérience liée à l'ADN et à l'ARN est venu des sociétés spécialisées dans la fourniture de produits pour la recherche et les applications en microbiologie et biologie moléculaire. Ces sociétés, en fournissant appareillages et ensembles tout agencés contenant tous les élements nécessaires pour effectuer les opérations expérimentales même les plus complexes en biologie ont grandement contribué à démocratiser ces technologies. Ces sociétés sont promptes à relayer auprès de leur client biologiste toute amélioration technologique jugée bénéfique découverte dans un laboratoire de la communauté scientifique et médicale mondiale. A ce jour aucune d'elles ne propose les produits et la méthologie qui font l'objet de cette invention.

Les différentes étapes de transformation d'une bactérie ou de microorganismes eucaryotes tels que les levures et champignons filamenteux par un ADN vecteur sont dans les grandes lignes les suivantes : les cellules réceptrices rendues aptes à incorporer l'ADN par divers traitements sont mises en contact avec l'ADN puis sont mises en culture dans un milieu liquide pour permettre l'apparition du caractère de sélection avant d'être étalées dans des boites de Pétri renfermant un milieu solide additionné de l'agent sélectif. A ce stade un substrat chromogénique ou fluorescent peut aussi être ajouté pour les vecteurs de clonage ou de transfert permettant ce moyen d'identification des colonies. Après une nuit d'incubation ou plusieurs jours selon la vitesse de croissance de l'organisme considéré, les cellules ayant initialement récupéré le vecteur et exprimant les transgènes vont former des colonies. Ces colonies sont ensuite récupérées pour préparer des cultures cette fois en milieux liquides contenant l'agent de sélection dans le but d'extraire l'ADN extrachromosomique qui sera ensuite caractérisé sur la base de la taille, la présence de sites d'enzymes de restriction et éventuellement par séquençage. Le volume de culture liquide sera fonction de la quantité d'ADN recherché par l'expérimentateur et pourra varier de quelques millilitres à un litre ou plus. Ces technologies lorsqu'elles sont appliquées aux microorganismes comme *E.coli* et *S.cerevisiae* par exemple sont parfaitement connues de l'homme de l'art et leur degré de difficulté ne représente en aucune facon une limitation pour la mise en oeuvre de l'invention.

L'invention porte sur un nouveau moyen de préparer des milieux liquides et solides sélectifs pour la sélection et la manipulation de microorganismes recombinés. Les milieux sélectifs sont actuellement préparés de façon identique par tous les biologistes expérimentateurs du monde entier. Les ingrédients entrant dans la composition d'un milieu donné sont mélangés à de l'eau dans un récipient de différentes formes le plus souvent en verre, fermé soit par un bouchon formé de coton, de mousse en polyuréthane ou tout autre matière perméable à l'air pouvant supporter des températures de 125-130°C et entouré par du papier aluminisé; soit fermé par un bouchon vissé en bakelite ou autre matière résistante. Le récipient est ensuite mis dans un autoclave pour stérilisation généralement pendant 20 min à 120°C. Après autoclavage le récipient est mis à refroidir jusqu'à une température de 40-50°C et à ce moment seulement l'antibiotique en solution stérile ainsi qu'éventuellement une solution stérile d'un substrat chromogénique ou fluorescent et une molécule inductrice de l'expression d'un transgène sont ajoutés aseptiquement au milieu contenu dans le récipient. Ces différents additifs ne peuvent être inclus dans les ingrédients du milieu avant autoclavage par suite de leur sensibilité à la température atteinte dans l'autoclave et de ce fait sont seulement ajoutés sous forme de solution stérilisée par filtration sur membrane dans le milieu après passage à l'autoclave. Dans le cas d'un milieu renfermant de la gélose les boites sont immédiatement préparées par répartition du milieu en surfusion dans des boites de Pétri stériles.

Les trois phases du cycle de stérilisation dans l'autoclave, à savoir la montée en température, la durée du maintien de la température désirée puis la descente en température demande un temps minimun d'une heure. L'accomplissement des autres tâches du procédé c'est à dire la préparation du milieu avant autoclavage, l'attente du refroidissement du milieu après ouverture de la porte de l'autoclave, le rajout des additifs et la préparation des boites de Pétri occupe encore au moins une autre heure, portant la durée totale de l'opération à environ deux heures. Si l'on rajoute le problème rencontré avec la disponibilité de l'autoclave, appareil volumineux bien souvent unique dans un lieu de travail, pas toujours libre au moment où un expérimentateur en a le plus besoin et les causes d'oublis et d'erreurs engendrées par les rajouts d'antibiotiques et autres molécules au milieu, on arrive à un procédé pourtant jusqu'à présent incontournable mais qui représente une sérieuse limitation pour une meilleure efficacité du temps de travail de l'expérimentateur. Une première solution apportée à ce niveau est la centralisation de la préparation des milieux par une personne ou un groupe de personnes qui assure la fourniture des milieux de cultures à un ensemble d'utilisateurs. Cette situation ne peut évidemment se rencontrer que dans les grandes unités. Une deuxième possibilité est l'achat des milieux liquides prêts à l'emploi et des boites de Pétri prêtes à l'emploi, solution qui serait parfaite si ce n'était le coût, facteur limitant pour les budgets d'un grand nombre de laboratoires et d'équipes du secteur public et privé.

L'utilisation des fours à microondes est devenue une pratique courante pour porter à bonne température des préparations culinaires conservées à température ambiante, au froid ou sous forme congelée. Les avantages des microondes sur les procédés traditionnels d'échauffement des aliments portent sur la rapidité et la simplicité d'exécution. La forte demande du marché a conduit à une banalisation des fours à microondes qui maintenant trouvent leur place dans les cuisines au même titre qu'un réfrigérateur. Une grande diversité d'appareils se distinguant par la puissance des ondes, le volume intérieur et les possibilités de programmation est maintenant disponible sur le marché des arts ménagers. Les fours à microondes ont également acquis depuis des années une place dans les laboratoires pour une application spécifique à la biologie moléculaire. La caractérisation de la taille des acides nucléiques est faîte par des mesures de distances de migrations après électrophorèse en gel d'agarose. L'agarose, agent solidifiant du gel est une forme purifiée de la gélose employée pour solidifier les milieux de culture. Une technique maintenant bien répandue consiste à faire fondre l'agarose additionné du tampon aqueux dans un four à microondes afin de pouvoir couler la solution en surfusion dans la cuve de l'appareil à électrophorèse pour former le gel. La stérilité du gel n'est absolument pas recherchée pour cette application et seule la rapidité a conduit à la généralisation de cette technique par opposition à celle faisant intervenir la fusion de l'agar dans un autoclave.

Il serait extrêmement avantageux de pouvoir substituer l'emploi d'un autoclave par celui d'un four à microondes pour la préparation des milieux de cultures liquides et solides. Seulement la condition primordiale est ici que les milieux doivent être entièrement stériles c'est à dire dépourvus de microorganismes. C'est parce qu'il a toujours été considéré que les fours à microondes ne pouvaient assurer une stérilité suffisante que ceux ci n'ont jamais été préconisés pour la préparation de milieux de cultures pour les microorganismes. Une confirmation de cette affirmation est trouvée dans le livre de base de tout microbiologiste (Handbook of Microbiological Media, second edition,1997, by Ronald M.Atlas. Edited by Laurence C .Parks CRC Press) qui référence des milliers de milieux en donnant leur composition et leur mode de préparation mais qui pas une seule fois ne mentionne l'utilisation des fours à microondes en place des autoclaves.

L'action des microondes exercée pendant des temps compatibles avec l'utilisation recherchée n'est pas suffisamment microbiocide pour tuer tous les microorganismes présents dans les ingrédients solides des milieux. Les ingrédients des milieux constitués par des matières organiques d'origine animale, végétale ou microbienne et des matières minérales renferment à des degrés divers une grande diversité de microorganismes.

Un des moyens de permettre l'obtention de milieux stériles par microondage est de stériliser les matières premières entrant dans la composition des milieux avant le passage aux microondes. Le mélange en poudre de chacun des constituants des milieux peut être stérilisé par exemple, par irradiation gamma dans une installation adaptée existant dans les établissements équipés pour la stérilisation par irradiation de produits alimentaires. Le maintien de la stérilité des poudres avant utilisation est assuré par répartition aseptique de la poudre irradiée dans des sachets stériles à raison d'une quantité unitaire de poudre suffisante pour préparer un volume donné de milieu. La fabrication de milieux de cultures remplissant les critères requis de stérilité se fait donc à partir de ces sachets dans les conditions suivantes: un récipient stérile fermé par un bouchon vissé non métallique dans lequel le contenu d'un sachet correspondant à un milieu donné a été versé et mélangé avec le volume d'eau stérile nécessaire est placé dans un four à microondes. Le récipient est soumis aux actions des microondes pendant un temps d'environ trois minutes au maximun de puissance de l'appareil avec deux ou trois arrêts régulièrement espacés de quelques secondes pour agiter par rotation le récipient afin de faciliter la dissolution uniforme des poudres et la fonte de l'agar dans le cas des milieux solides. Après 5 à 10 min de refroidissement, l'antibiotique correspondant et autres additifs sont ajoutés pour donner un milieu liquide prêt à l'utilisation ou un milieu solide prêt à couler pour la fabrication des boites de Pétri.

Une encore plus grande simplification du protocole de préparation des milieux par l'utilisation des microondes serait que les agents de sélection et d'identification soient déja présents dans le contenu du sachet. Un certain nombre de ces additifs se sont avérés sensibles aux températures générées par les microondes et ne peuvent de ce fait être inclus dans les poudres.

Cependant au cours des très nombreux essais de mise au point de cette nouvelle technique, la Société Demanderesse a trouvé que des constituants entrant dans la composition de certains milieux exerçaient un effet thermoprotecteur sur des additifs sensibles à la chaleur comme les antibiotiques du type penicilline ou l'X-gal par exemple. Des substances reconnues pour leur effet de cryoprotection et de protection à la lyophilisation telles le saccharose, le tréhalose, les amylopectines, divers amidons ainsi que le mannitol et le sorbitol se sont avérées à des degrés divers augmenter la résistance de molécules à l'inactivation par la chaleur générée par les microndes dans des solutions aqueuses. Grâce à l'addition d'une de ces substances stabilisatrices dont le choix et la concentration doit tenir compte de l'absence d'effet sur la croissance du microorganisme étudié, la présente invention permet d'inclure la plupart des additifs de sélection et d'identification des microorganismes recombinés dans les sachets, supprimant ainsi une étape post microondage.

Cette découverte a de plus fourni un nouveau procédé pour obtenir la stérilité de milieux soumis à l'action des microondes. En effet, la stérilité est obtenue par l'addition, aux mélanges en poudre non stérilisés des ingrédients du milieu et d'une substance thermoprotectrice, de substances toxiques pour les microorganismes autres que celui devant être cultivé dans le milieu à préparer. Dans le cas d'*E.coli*, bactérie gram négative, l'addition d'antibiotiques spécifiquement actifs sur les bactéries gram positives et de substances antifongiques assure avec l'addition de l'antibiotique de sélection des transformants, une stérilité parfaite après passage aux microondes sans qu'il soit nécessaire de recourir à la stérilisation des poudres par l'opération efficace mais onéreuse de l'irradiation gamma.

Dans le cas des levures, l'addition d'un antibiotique à large spectre d'activité antibactérienne et de l'antibiotique de sélection des transformants de la levure tel la zéocine assure une stérilité parfaite et une croissance normale des colonies.

Le document D1 (Tisserat et al.) considéré comme l'art antérieur le plus proche, décrit la stérilisation par microondes de milieu de culture de plantes, sans incorporation d'agents de sélection et d'identification avant soumission à l'action de microondes.

### Exemple 1 :

L'exemple suivant est illustratif de toute une série d'expériences dont les résultats aboutissent à la même observation qui est que le dégagement de chaleur provoquée par l'action des microondes sur un milieu aqueux n'est pas suffisant pour assurer une stérilité des milieux compatible avec une utilisation en microbiologie et biologie moléculaire.

Les constituants en poudre du milieu classique LB pour *E.coli* nécessaires à la préparation de 200 ml de milieu dont la composition est trouvée dans le Handbook of Microbiological Media page 743 : extrait de levure Bio Springer 1g, hydrolysat pancréatique de caseine Difco 2g, NaCl Merck 1g, gélose Difco 3g) sont pesés de facon précise et mélangés dans deux flacons en verre de 500ml fermés par un bouchon vissé. La même quantité de poudre du milieu LB préconstitué (7g) commercialisé par BIO101 est ajoutée dans deux autres flacons Après ajout de 200ml d'eau bidistillée, deux flacons sont autoclavés ensemble à 120°C pendant 20 min alors que les deux autres flacons sont passés séparément dans un four à microondes réglé à puissance moyenne pendant une fois 3 min, puis deux fois 30 secondes chaque fois après avoir été agité pendant quelques secondes. Les milieux après les traitements thermiques présentent le même aspect limpide de couleur jaune paille. L'ampicilline en solution stérile est alors ajoutée à chaque flacon à la concentration finale de 50 µg/ml et neuf boites sont alors coulées à partir de chacun des milieux gélosés. Une culture de la souche DH5 d'*E.coli* est préparée à partir de cellules compétentes mises en contact avec l'ADN du plasmide pBR322 dans du milieu LB liquide pendant 90 min. Une fraction de la culture est étalée sur 3 boites provenant de chacun des milieux dans une hotte à flux laminaire stérile. Les boites sont ensuite incubées à 37°C. Trois boites non ensemencées sont également incubées à 37°C pendant 48h et les trois autres à 27°C pendant 7 jours. Les résultats sont présentés dans le tableau 1.

**Tableau 1**

| | Milieu autoclavé | | Milieu microondé | |
|---|---|---|---|---|
| | Non ensemencé | +DH5 Nombre de colonies | Non ensemencé | + DH5 Nombre Nombre de colonies |
| Milieu Reconstitué | 37°C stérile | 200 (e) | 37°C a | F |
| | 37°C stérile | 177 (e) | 37°C a | F |
| | 37°C stérile | 209 (e) | 37°C a | F |
| | 27°C stérile | | 27°C b | |
| | 27°C stérile | | 27°C b | |
| | 27°C stérile | | 27°C b | |
| Milieu Préconstitué | 37°C stérile | | 37°C c | |
| | 37°C stérile | | 37°C c | |
| | 37°C stérile | | 37°C c | |
| | 27°C stérile | | 27°C d | |
| | 27°C stérile | | 27°C d | |
| | 27°C stérile | | 27°C d | |
| a- Les boites renferment une moyenne de 30 colonies de diverses tailles (grosseur d'une tête d'épingle jusqu'à 3 mm) et de couleurs différentes (translucides à jaune brun) indiquant une hétérogénéité de microorganismes contaminants. b- Les boites sont entièrement couvertes par un mycelium hétérogène de deux champignons , l'un de couleur blanche et l'autre de couleur verte. c- Les boites renferment une dizaine de colonies dont la majorité homogène est de 2 mm et de couleur ambrée. d- Les boites renferment une vingtaine de grosses colonies blanches attribuées à des levures enfouies sous un mycelium blanc recouvrant les boites en entier. e-Les boites contiennent uniquement des colonies d'E.coli. f-Les boites contiennent des colonies d'E.coli mélangées à des colonies d'organismes contaminants | | | | |

Les résultats de la manipulation décrite dans cet exemple ont été reproduits chaque fois que l'expérience a éte faite. De plus il a été trouvé que le nombre et le type de contaminants microbiens observés avec les milieux microondés variaient fortement selon les lots des matières premières entrant dans la composition du milieu de sélection provenant d'un même fournisseur ou de fournisseurs différents.

### Exemple 2 :

Cet exemple montre qu'un traitement répété de chauffage d'un milieu aqueux non-stérile par les microondes peut cependant conduire à la stérilité de ces milieux par opposition à un traitement unique.

Une première série A constituée par 18 flacons de 500 ml renfermant 4 g du mélange reconstitué de poudres du milieu LB non gélosé (extrait de levure Difco 1g, biotrypcase Bio-Merieux 2g, NaCl Prolabo 1g ) et 200 ml d'eau du robinet est préparée. Une deuxième série B constituée par 18 flacons contenant 7g du mélange reconstitué de poudres du milieu LB gélosé (extrait de levure Difco 1g, hydrolysat pancréatique de caseine Bio Merieux 2g, NaCl -Prolabo 1g, gélose Biokar 3g) et 200 ml d'eau du robinet est préparée. Six flacons de la série A et six flacons de la série B sont autoclavés à 120°C pendant 20 min. Chacun des flacons restants des deux séries est passé dans un four à microondes réglé au maximun de puissance pendant une fois 2 min, puis deux fois 30 secondes chaque fois après avoir été lentement agité pendant quelques secondes. Une moitié des flacons de chaque série traité une fois aux microndes est retraité une deuxième fois selon le même protocole que précedemment après un intervalle de temps d'au moins une heure entre les deux traitements de facon à permettre au milieu non gélosé de se refroidir completement et aux milieux gélosés de se prendre en masse dans le flacon. Les flacons de la série A autoclavés et microondés une fois ou deux fois sont mis à incuber sans agitation à 37°C et 27°C. Les boites sont coulées à partir des milieux gélosés et mises à incuber dans des étuves à 37°C pendant 3 jours et à 27°C pendant 7 7 jours. Les résultats sont présentés dans le tableau 2 et le Tableau 3.

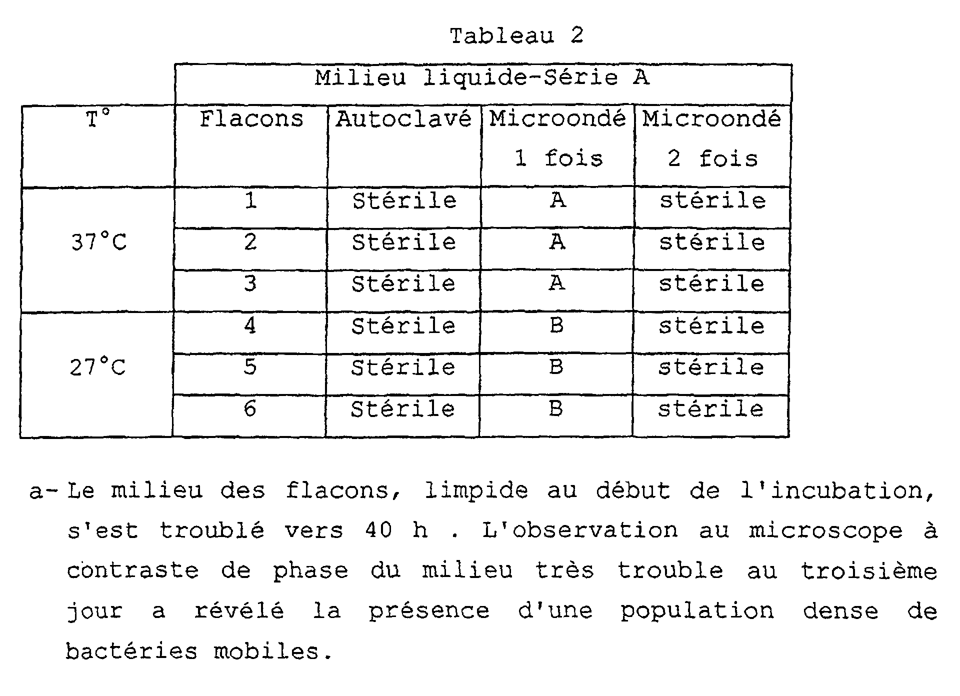

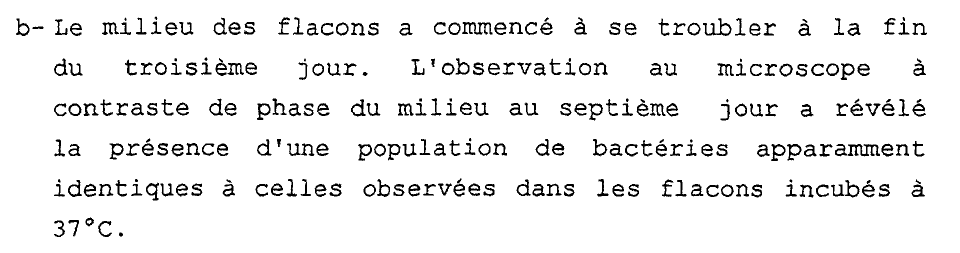

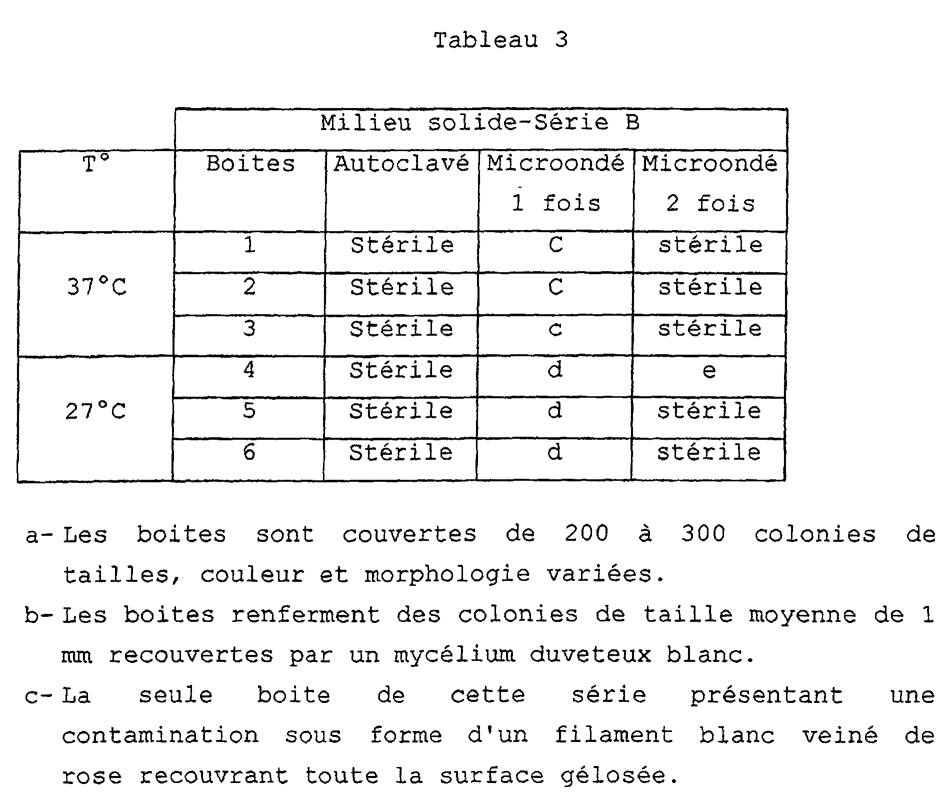

### Exemple 3 :

L'exemple suivant démontre que la stérilité des milieux microondés est obtenue lorsque les poudres entrant dans la composition des milieux de culture à reconstituer avec de l'eau sont elles-même stériles.

Un échantillon de 6kg du mélange de poudres entrant dans la composition du milieu de culture Terrific Broth développé pour l'obtention de cultures denses d'E.coli et bien connu des biologistes moléculaires dont la composition est trouvée dans le Handbook of Microbiological Media page 1356 est préparé en une seule fois (extrait de levure Bio Springer 3024g, tryptone Difco 1512g, KH2PO4 Prolabo 1184g , K2HPO4 Prolabo 277g). Une fraction importante (5kg) est stérilisée par irradiation gamma dans une installation utilisée pour la stérilisation de produits alimentaires destinés à la consommation humaine (GAMMASTER France). La totalité de l'échantillon irradié est ensuite répartie manuellement selon les conditions expérimentales d'aseptie pratiquée par tout microbiologiste dans une enceinte à flux laminaire, dans des sachets aluminisés stériles à raison de 10 g par sachet. Les sachets sont ensuite scellés par thermosoudure à l'aide d'un appareil approprié et conservés à température ambiante dans une boite cartonnée.

Le contenu de chaque sachet renfermant de la poudre soumise à irradiation gamma ou le même mélange de poudre non-irradiée est versé dans un flacon de 500 ml et repris par 200 ml d'eau distillée. Chaque flacon est ensuite microondé à moyenne puissance pendant 3 min puis deux fois 20 sec. Après refroidissement, 75 ml de milieu prélevés en condition aseptique à partir de chaque flacon sont répartis dans trois erlenmeyers stériles fermés par un bouchon en mousse. Les erlenmeyers sont mis à agiter sur une table agitante dans une chambre thermostatée à 37°C pendant une semaine. L'apparition de contaminants microbiens est suivie tous les jours par observation visuelle de la turbidité du milieu de chaque flacon et l'observation au microscope de prélèvements réalisés à partir de chaque flacon dont le contenu est devenu trouble. Les résultats sont présentés dans le tableau 4.

**Tableau 4**

| Flacon | Erlen | Poudre irradiée | Poudre non irradiée |
|---|---|---|---|
| 1 | 1 | stérile | b |
| | 2 | stérile | b |
| | 3 | stérile | b |
| 2 | 1 | stérile | b |
| | 2 | stérile | b |
| | 3 | stérile | b |
| 3 | 1 | a | b |
| | 2 | stérile | b |
| | 3 | stérile | b |
| a- Le seul erlenmeyer de cette série qui a été contaminé à partir du troisième jour. b- Le milieu de tous les erlenmeyers de cette série est devenu trouble entre la fin du premier et du deuxième jour d'incubation. | | | |

Des tests identiques de stérilité après passage aux microondes ont été répétés trois fois, pendant une période de plusieurs semaines, avec les poudres des sachets restants. Les résultats se sont avérés concordants avec les résultats présentés dans le tableau 4, les milieux préparés à partir des poudres stérilisées sont restés stériles durant toute la durée d'incubation alors que la totalité des flacons contenant le milieu préparé à partir des poudres non traitées développait rapidement une contamination de nature bactérienne.

### Exemple 4

Cet exemple montre que la stérilité des milieux spécifiques pour la sélection des colonies transformées d'*E.coli* pour la résistance à l'ampicilline peut aussi être obtenue par addition d'antibiotiques en poudre dans le mélange des ingrédients avant mise en dissolution dans l'eau par l'action des microondes.

On prépare un échantillon de 1050g du mélange des constituants en poudre entrant dans la composition du milieu LB gélosé dont la recette est trouvée dans le Handbook of Microbiological Media (page 743) : extrait de levure Bio Springer 150g, hydrolysat pancréatique de caseine Biokar 300g, NaCl Prolabo 150g, gélose Difco 450g auquel on ajoute 150g d'amylopectine Roquette Frères. L'échantillon est divisé en quatre lots égaux de 300 g désignés lot A, lot B, lot C et lot D. Au lot B, on ajoute 750 mg d'ampicilline Sigma en poudre. Au lot C, on ajoute 750 mg d'ampicilline Sigma en poudre et 75 mg d'acide fusidique Leo. Au lot D, on ajoute 750 mg d'ampicilline Sigma en poudre, 75mg d'acide fusidique Leo et 75 mg de nystatine Sigma. Les mélanges bien homogénéisés des poudres des lots A,B,C et D sont répartis à raison de 8g dans des sachets aluminisés de dimensions 5cm sur 9.5cm qui sont ensuite scellés par soudure à chaud.

Deux flacons de 500 ml sont préparés pour chaque lot en versant le contenu d'un sachet qui est remis en suspension dans 200 ml d'eau du robinet. Un flacon de chaque lot est autoclavé pendant 20 min à 120°C puis 9 boites sont coulées. Le deuxième flacon de chaque lot est microondé dans un four à microondes à puissance maximale pendant 2 min et deux fois trente secondes, durées entrecoupées par une agitation lente du flacon hors microondes pendant quelques secondes pour faciliter une homogénéisation du milieu puis 9 boites de Pétri sont coulées.

Une fraction diluée d'une culture de la souche compétente MC1061 d'*E.coli* mise en contact avec l'ADN du plamide pUC19 et après croissance en milieu LB pendant 60 min est étalée sur trois boites de chaque lot ayant été préparées par autoclavage et trois boites de chaque lot ayant été préparées par microondage. Les boites ensemencées sont incubées à 37°C pendant 48h alors que pour les six boites restantes non ensemencées pour chaque flacon, 3 boites sont incubées à 37°C pendant 3 jours et 3 boites sont incubées à 27°C pendant une semaine. Les résultats de l'état des boites après incubation sont présentés dans les tableaux 5 et 6.

**Tableau 5**

| Lots | Milieu autoclavé | | |
|---|---|---|---|
| | Boites non ensemencées | | MC1061 (pUC19) |
| | 37°C | 27°C | |
| Lot A | Stérile | stérile | voile |
| | | | d'E.coli |
| Lot B | Stérile | stérile | voile |
| | | | d'E.coli |
| Lot C | Stérile | stérile | voile |
| | | | d'E.coli |
| Lot D | Stérile | stérile | voile |
| | | | d'E.coli |

Les résultats de ce tableau indiquent clairement que le passage par l'autoclave a détruit les activités des antibiotiques rajoutés dans les différents lots de milieux puisque un voile uniforme des cellules non transformées de la souche MC1061 recouvre toutes les boites.

**Tableau 6**

| Lots | Milieu microondé | | |
|---|---|---|---|
| | Boites non ensemencées | | MC1061 (pUC19) |
| | 37°C | 27°C | |
| Lot A | 30 à 50 colonies de diverses tailles | Mycélium | voile d'E.coli |
| Lot B | 10 colonies de 1 mm | Mycélium | Colonies d'E.coli + colonies d'organismes contaminants |
| | 2 colonies de 5 mm duveteuses | | |
| Lot C | 3 à 5 colonies de 5 mm duveteuses | Mycélium | 118-145-190 colonies d'E.coli + 3 colonies duveteuses |
| Lot D | Stérile | Stérile | 125-150-160 colonies d'E.coli |

Les résultats de ce tableau démontrent que le milieu du lot D contenant l'ampicilline, antibiotique de sélection des transformants, l'acide fusidique, antibiotique actif sur les bactéries à grain positif et la nystatine, substance antifongique active sur levures et champignons filamenteux donne, après passage aux microondes, des milieux solides parfaitement stériles et compatibles avec une sélection optimale des colonies de cellules transformées. En effet, le nombre des colonies apparues dans les boites du milieu D microondé est identique à celui observé sur des boites, préparées par la méthode conventionnelle de stérilisation du milieu sélectif et ajout de l'ampicilline après autoclavage, ensemencées avec le même nombre de cellules de la souche MC1061. D'autre part l'ADN du plasmide pUC19 est retrouvé dans tous les clones préparés à partir des colonies prélevées sur les boites du milieu D.

### Exemple 5 :

Cet exemple illustre la méthode de préparation d'un milieu standard d'*E*. *coli* pour la sélection de colonies résistantes à l'ampicilline et de coloration bleue ou blanche selon la présence ou non d'une activité β-galactosidase basée sur l'utilisation de poudres préconditionnées à mettre en suspension dans l'eau et à dissoudre par l'intermédiaire d'un four à microondes. Les avantages de cette méthode en termes de simplicité et de temps sont clairement montrés par comparaison à la méthode traditionnelle.

Un échantillon d'environ 52kg de poudre formé par le mélange des ingrédients suivants: extrait de levure pauvre en hydrates de carbone Biokar 6250g, tryptone Biokar 12500g, amylopectine Roquette Frères 2500g, NaCl Compagnie des Salins 6250g, Na2HPO4 Prolabo 6250g, gélose Sobigel 18750g, ampicilline Sigma 125g, acide fusidique Leo 12,5g, nystatine Sigma 12,5g, IPTG Biosynth 125g, X-gal Biosynth 62,5g, est réparti en sachets aluminisés de 5,5 cm x 9,5 cm dans une chaîne automatique de conditionnement (Lallemand). Les sachets portant la dénomination Fast-Blue Amp pour la moitié d'entre eux et Fast X-Gal Amp pour l'autre moitié sont remplis avec 8 g de poudre et scellés le long de la chaîne sans intervention manuelle. Fast-Blue et Fast X-Gal sont deux désignations différentes pour le même produit qui ont fait l'objet d'un dépot de marque en France. Les sachets ont tous été utilisés dans les services microbiologie et thérapie génique de la société Cayla afin d'évaluer sur une grande échelle leur intérêt pour la préparation de boites de milieu sélectif pour l'isolement de clones *d'E.coli* transformés par divers plasmides natifs ou provenant de produits de ligation lors d'expériences de clonage de gènes.

Une utilisation type des produits Fast-Blue Amp et Fast X-Gal Amp est la suivante:

Le contenu d'un sachet est versé dans un récipient en verre ou plastique résistant à des températures supérieures à 100°C de formes quelconques et d'une capacité voisine de 500 ml, c'est à dire compatible avec les dimensions intérieures de l'appareil à microondes utilisé et repris par 200 ml d'eau dont la qualité minimale requise est d'être potable. La suspension est chauffée sous l'action des microondes en prenant comme seule précaution de ne pas dépasser le stade de l'ébullition qui conduirait à un débordement du milieu hors du flacon. Cette condition est facilement remplie en soumettant le récipient à des flux d'ondes courts et répétés entrecoupés par des phases brèves d'agitation du récipient pour faciliter la dissolution homogène des ingrédients du récipient maintenu avec des gants de protection contre la chaleur. Cette opération de microondage proprement dite est achevée en moins de 5 min. Les boites sont ensuite remplies après une attente de 5 à 10 min pour permettre le refroidissement du contenu du récipient jusqu'à une température plus confortable pour les mains de l'utilisateur même ganté. La durée totale de préparation des boites par cette technologie du début de l'opération avec la prise du sachet jusqu'à la disponibilité de 8 à 10 boites de Pétri n'excède pas 25 min à comparer avec les 2 h nécessaires par la méthode habituelle passant par un autoclavage du milieu. D'autre part, le nombre de phases d'intervention de l'expérimentateur est réduit toujours par comparaison avec la méthode traditionnelle, diminuant ainsi considérablement les possibilités d'erreurs fréquemment constatées lors de l'addition de l'antibiotique de sélection et autres substances après autoclavage.

L'utilisation en interne à Cayla des milliers de sachets Fast-Blue et Fast X-gal a permis de démontrer la qualité des milieux préparés par cette méthodologie pour la sélection des souches transformées d'*E.coli*. Les expériences de comparaison entre les milieux solides préparés à partir des produits Fast et les microondes et la méthode traditionnelle ont toutes démontré que le temps nécessaire à l'apparition de la couleur bleue des colonies et l'intensité de la couleur étaient comparables sinon plus favorable pour la méthodologie faisant l'objet de cet invention. Par ailleurs la taille et le nombre des colonies apparues sur les boites étaient en tous points identiques dans les deux méthodes. Cette nouvelle technologie a été validée avec toutes les souches réceptrices d'E.coli testées et une très large variété de vecteurs plasmidiques permettant la distinction blanc-bleue des colonies.

### Exemple 6 :

Cet exemple montre que la méthode de préparation par les microondes de milieux de culture sélectifs pour des bactéries transformées s'applique également à la sélection de microorganismes recombinés eucaryotes.

On prépare un échantillon d'environ 1 kg du milieu YEPD dont la composition est décrite dans le Handbook of Microbiological Media (page 1573) : extrait de levure Bio Springer 150g, mycopeptone Biokar 300g, dextrose Roquette 300g, gélose Sobigel 225g auquel on ajoute Na2HPO4 Merck 45g, ampicilline Sigma 1,5g, zéocine Invitrogen 0,75g. On introduit 14g de ce mélange de poudres dans un erlenmeyer de 500ml et 200ml d'eau bidistillée puis le flacon est passé dans un four à microondes réglé à moyenne puissance pendant 2 min , 1 min et 30 secondes avec agitation dans les deux intervalles pendant 5 secondes par rotation hors de l'appareil. Le milieu en surfusion est ensuite réparti dans des boites de Pétri à raison de 25 ml par boite.

Une culture d'une souche de laboratoire de la levure *Saccharomyces cerevisiae* OL1 *(mat a leu2-3 leu2-112 his3-11 his3-15 ura3-251 ura3-373*) est rendue compétente par la méthode au lithium bien connue des hommes de l'art et après contact avec l'ADN du plasmide pUT332 (Cayla) pendant 30 min est diluée au 1/10 dans du milieu YEPD liquide. La culture est incubée sous agitation à 30°C pendant 6 heures et après refroidissement dans de la glace pilée pendant une heure une fraction est étalée sur les boites du milieu sélectif microondé et des boites préparées par la méthode traditionnelle de l'autoclave de milieu YNB (Handbook of Microbiological Media, page 1568) supplémenté avec du glucose, de la leucine. et de l'histidine. Après 3 jours d'incubation à 30°C le nombre de colonies apparues dans les boites est identique avec les deux milieux, la taille des colonies sur le milieu riche microondé étant le double de celle des colonies apparues sur le milieu défini. Les boites ensemencées avec la culture contrôle n'ayant pas été en contact avec l'ADN transformant sont restées vierges de colonies aussi bien avec le milieu riche microondé que le milieu défini autoclavé. La vérification du phénotype des colonies apparues sur le milieu microondé a montré qu'il correspondait bien à celui attendu pour les transformants du plasmide pUT 332, c'est à dire zeo^{r} (résistance à la zéocine) et ura⁺ (capacité à pousser sur un milieu dépourvu d' uracile).

## Revendications

1. Procédé de préparation d'un milieu de culture stérile pour la sélection de microorganismes recombinés, **caractérisé par le fait que**:
- l'on prépare un mélange sous forme de poudre de tous les ingrédients du milieu, y compris les agents de sélection et d'identification et d'une substance themoprotectrice
- l'on dissout la poudre dans une quantité suffisante d'eau,
- l'on soumet la suspension ainsi obtenue à l'action de microondes afin de l'homogénéiser.

2. Procédé selon la revendication 1, **caractérisé par le fait que** le mélange sous forme de poudre est préalablement stérilisé, de préférence par soumission à une irradiation aux rayonnements gamma.

3. Procédé selon la revendication 1, **caractérisé par le fait que** l'on ajoute, outre les agents de sélection et d'identification, éventuellement d'autres additifs d'induction au mélange sous forme de poudre.

4. Procédé selon la revendication 3, **caractérisé par le fait que** l'agent de sélection est un antibiotique choisi dans le groupe des penicillines, les aminosides, la tétracycline, le chloramphénicol et la zéocine.

5. Procédé selon la revendication 3, **caractérisé par le fait que** l'agent d'identification est une molécule chromogénique ou fluorescente.

6. Procédé selon la revendication 3, **caractérisé par le fait que** la molécule inductrice de l'expression d'un transgène est un sucre simple ou modifié comme l'IPTG.

7. Procédé selon la revendication 3, **caractérisé par le fait que** le milieu de culture est destiné aux bactéries à gram négatif comme *Escherichia coli* et que l'on ajoute au moins un antibiotique spécifique des bactéries à gram positif au mélange sous forme de poudre.

8. Procédé selon la revendication 3, **caractérisé par le fait que** l'on ajoute une molécule à activité antifongique au mélange sous forme de poudre.

9. Procédé selon la revendication 3, **caractérisé par le fait que** le milieu est destiné aux microorganismes eucaryotes comme les levures et que l'on ajoute au moins une molécule à activité antibactérienne à large spectre au mélange sous forme de poudre.

10. Procédé selon la revendication 3, **caractérisé par le fait que** l'on ajoute au moins une substance reconnue pour son activité de protection à la congélation ou à la lyophilisation telle que le saccharose, le tréhalose, les amylopectines, les amidons, le mannitol ou le sorbitol au mélange sous forme de poudre.

11. Procédé selon l'une quelconque des revendications 1 à 9, **caractérisé par le fait que** l'action des microondes est fournie par un four à microondes.

## Patentansprüche

1. Verfahren zur Herstellung eines sterilen Kulturmediums für die Auswahl von rekombinierten Mikroorganismen, **dadurch gekennzeichnet, daß**:
- ein Gemisch in Pulverform aus allen Bestandteilen des Mediums, wobei darin die Mittel zur Auswahl und zur Identifizierung eingeschlossen sind, und einer Wärmeschutzsubstanz hergestellt wird,
- das Pulver in einer ausreichenden Wassermenge aufgelöst wird,
- die so erhaltene Suspension einer Mikrowellenbehandlung unterzogen wird, um sie zu homogenisieren.

2. Verfahren nach Anspruch 1, **dadurch gekennzeichnet, daß** das Gemisch in Pulverform vorher sterilisiert wird, vorzugsweise durch Unterziehung einer Bestrahlung mit Gammastrahlen.

3. Verfahren nach Anspruch 1, **dadurch gekennzeichnet, daß** außer den Mitteln zur Auswahl und zur Identifizierung unter Umständen weitere Induktionszusätze dem Gemisch in Pulverform hinzugefügt werden.

4. Verfahren nach Anspruch 3, **dadurch gekennzeichnet, daß** das Auswahlmittel ein Antibiotikum ist, das aus der Gruppe der Penicilline, der Aminoside, des Tetracyclins, des Chloramphenicols und des Zeocins gewählt ist.

5. Verfahren nach Anspruch 3, **dadurch gekennzeichnet, daß** das Identifizierungsmittel ein farbbildendes oder fluoreszierendes Molekül ist.

6. Verfahren nach Anspruch 3, **dadurch gekennzeichnet, daß** das Induktionsmolekül des Ausdrucks eines Transgens ein einfacher oder modifizierter Zucker wie z.B. IPTG ist.

7. Verfahren nach Anspruch 3, **dadurch gekennzeichnet, daß** das Kulturmedium für gramnegative Bakterien wie z.B. Escherichia coli bestimmt ist und daß wenigstens ein spezifisches Antibiotikum von grampositiven Bakterien dem Gemisch in Pulverform hinzugefügt wird.

8. Verfahren nach Anspruch 3, **dadurch gekennzeichnet, daß** ein Molekül mit antimykotischer Wirkung dem Gemisch in Pulverform hinzugefügt wird.

9. Verfahren nach Anspruch 3, **dadurch gekennzeichnet, daß** das Medium für eucaryote Mikroorganismen wie z.B. Hefepilze bestimmt ist und daß wenigstens ein Molekül mit breitbandiger antibakterieller Aktivität dem Gemisch in Pulverform hinzugefügt wird.

10. Verfahren nach Anspruch 3, **dadurch gekennzeichnet, daß** wenigstens eine für ihre Schutzaktivität bei der Einfrierung oder bei der Gefriertrocknung bekannte Substanz wie z.B. Saccharose, Trehalose, Amylopectine, Stärken, Mannit oder Sorbit dem Gemisch in Pulverform hinzugefügt wird.

11. Verfahren nach einem der Ansprüche 1 bis 9, **dadurch gekennzeichnet, daß** die Mikrowellenbehandlung durch einen Mikrowellenherd erbracht wird.

## Claims

1. Process for the preparation of a sterile culture medium for the selection of recombinant microorganisms, **characterized in that** :
- a mixture, in the form of powder of all the ingredients of the medium, including selection and identification agents and a thermoprotective substance, is prepared,
- the powder is dissolved in a sufficient quantity of water,
- the resulting suspension is subjected to the action of microwaves in order to homogenize it.

2. Process according to claim 1, **characterized in that** the mixture in the form of powder is sterilized beforehand, preferably by subjection to irradiation by gamma rays.

3. Process according to claim 1, **characterized in that** selection and identification agents, eventually other induction additives are further added to the mixture in the form of powder.

4. Process according to claim 3, **characterized in that** the selection agent is an antibiotic selected in the group of penicillins, aminosides, tetracyclin, chloramphenicol and zeocin.

5. Process according to claim 3, **characterized in that** the identification agent is a chromogenic or fluorescent molecule.

6. Process according to claim 3, **characterized in that** the inductive molecule of the expression of a transgene is a simple or modified sugar such as IPTG.

7. Process according to claim 3, **characterized in that** the culture medium is designed for gram negative bacteria such as Escherichia coli and **in that** at least one antibiotic that is specific to gram positive bacteria is added to the mixture in the form of powder.

8. Process according to claim 3, **characterized in that** a molecule having antifungal activity is added to the mixture in the form of powder.

9. Process according to claim 3, **characterized in that** the medium is designed for eucaryotic microorganisms such as yeasts and **in that** at least one molecule having a large spectrum antibacterial activity is added to the mixture in the form of powder.

10. Process according to claim 3, **characterized in that** at least one substance known to have protective activity against freezing or lyophilisation such as saccharose, trehalose, amylopectins, starches, mannitol or sorbitol is added to the mixture in the form of powder.

11. Process according to any one of claims 1 to 9, **characterized in that** the action of microwaves is provided by a microwave oven.
